# EUROPÄISCHE PATENTSCHRIFT

(11) **EP 0 811 834 B1**
(45) Veröffentlichungstag und Bekanntmachung des Hinweises auf die Patenterteilung: **29.08.2001**
(21) Anmeldenummer: 97109047.7
(22) Anmeldetag: 04.06.1997
(51) Int. Cl.: G01N 19/04, G01N 33/44, G01N 3/04

(54) **Verfahren und Vorrichtung zum Bestimmen der Grünklebrigkeit von Gummimischungen**
Method and device for determining green adhesion of rubber compounds
Méthode et appareil pour déterminer l'adhésion à vert de compositions de caoutchouc

(30) Priorität: 04.06.1996 DE 19622410
(43) Veröffentlichungstag der Anmeldung: 10.12.1997
(73) Patentinhaber: Pirelli Reifenwerke GmbH, 64747 Breuberg (DE)
(72) Erfinder: Hock, Bernd, 63762 Gross-Ostheim (DE)
(74) Vertreter: Preissner, Nicolaus, Dipl.-Ing.

(56) Entgegenhaltungen:
- DD-A- 283 900
- US-A- 2 762 219
- US-A- 2 775 888
- US-A- 3 372 583

## Beschreibung

Die Erfindung betrifft ein Verfahren und eine Vorrichtung zum Bestimmen der Grünklebrigkeit von Gummimischungen.

Die Klebekraft von Gummimischungen, die sogenannte Grünklebrigkeit, stellt für die Gummiindustrie eine wichtige Größe dar. So ist beispielsweise im Reifenbau die Grünklebrigkeit der Halbzeuge entscheidend dafür, ob man die jeweiligen Gummimischungen überhaupt miteinander verbinden kann. Somit stellt die Bestimmung der Grünklebrigkeit eine wichtige Grundlage für die Qualitätssicherung dar.

Die Grünklebrigkeit von Gummimischungen wird dabei durch Handmessung oder Kraftmessung bestimmt. Bei der Handmessung werden zwei Probestreifen der in Frage stehenden Gummimischungen mit der Hand zusammengedrückt und danach wieder abgezogen. Die von der diese Messung durchführenden Person subjektiv empfundene Abzugskraft wird dabei entsprechend einem Notensystem bewertet und eingestuft. Bei dieser Methode wird die Grünklebrigkeit somit durch Vergleich mit subjektiven Erfahrungswerten bestimmt. Bei der Kraftmessung wird ein Streifen aus Polyesterfolie (PE-Folie) mit einer Walze auf eine Probe der Gummimischung oder auf das Halbzeug aufgerollt und danach mit einem Kraftmesser horizontal abgezogen. Bei dieser Methode zur Bestimmung der Grünklebrigkeit wird zwar unter Verwendung eines Kraftmessers eine objektiv meßbare Kraft bestimmt, die zur Ablösung der Folie von der Gummimischung notwendig ist, jedoch sind die Randbedingungen einer solchen Kraftmessung nicht geeignet, eine Reproduzierbarkeit des Meßergebnisses zu gewährleisten. Dies hat zur Folge, daß bei einer Durchführung der Kraftmessung durch verschiedene Personen die Wahrscheinlichkeit sehr gering ist, daß übereinstimmende Meßwerte erzielt werden. Die Zuverlässigkeit solchermaßen gemessener Werte ist somit gering.

Die US 3 372 583 beschreibt ein Verfahren zur Kraftmessung der Grünklebrigkeit von Gummimischungen, bei dem zwei ebene Lagen der zu untersuchenden Gummimischung mit vorbestimmtem Druck während einer vorbestimmten Zeit zusammengepreßt werden, wobei sich zwischen den Lagen eine Polyesterfolie mit einer Ausnehmung befindet, die die Meßfläche, d.h. den Bereich, an dem die Lagen zusammenkleben, festlegt. Die Gummilagen werden auf zwei mit gleicher Geschwindigkeit in entgegengesetzter Richtung rotierende Scheiben aufgerollt und so getrennt. Die dabei auf die Achse einer der Scheiben wirkende Kraft ist ein Maß für die Abzugskraft.

Der Erfindung liegt die Aufgabe zugrunde, ein Verfahren zur Bestimmung der Grünklebrigkeit von Gummimischungen bereitzustellen, das eine hohe Reproduzierbarkeit und somit zuverlässige Meßwerte gewährleistet. Des weiteren liegt der Erfindung die Aufgabe zugrunde, eine Vorrichtung zur Bestimmung der Grünklebrigkeit von Gummimischungen bereitzustellen, die eine zuverlässige Bestimmung von Meßwerten mit einer hohen Reproduzierbarkeit gestattet.

Zur Lösung dieser Aufgabe wird erfindungsgemäß ein Verfahren zum Bestimmen der Grünklebrigkeit von Gummimischungen mit den Merkmalen des Anspruchs 1 vorgeschlagen.

Die Erfindung basiert auf der Erkenntnis, daß zum Verwirklichen einer reproduzierbaren Bestimmung der Grünklebrigkeit objektiv nachvollziehbare Randbedingungen vorliegen müssen. Dies wird zum einen dadurch erreicht, daß die für das Aufeinanderpressen der Gummimischungen, deren Grünklebrigkeit bestimmt werden soll, maßgeblichen Parameter, insbesondere die Größe des Drucks und die Länge der Zeitdauer, während der dieser Druck ausgeübt wird, sowie die Größe der aufeinander haftenden Meßflächen vorbestimmt werden. Der wesentliche Aspekt der Erfindung liegt jedoch darin begründet, daß die Meßflächen der aufeinanderzupressenden Gummiproben eine konvexe Wölbung aufweisen. Es wurde nämlich erkannt, daß zum Erreichen von reproduzierbaren Meßergebnissen auch Meßfehler eliminiert werden müssen, die aus Effekten herrühren, die beim Aufeinanderpressen und anschließenden Abziehen von Gummiproben auftreten und das Meßergebnis beeinflussen können. Dabei handelt es sich insbesondere um Saugeffekte zwischen den Meßflächen, die zur Folge haben, daß sich die gemessene maximale Abzugskraft nicht linear zur gemessenen Fläche verhält. Durch das erfindungsgemäße Vorsehen von konvex gewölbten Meßflächen werden beim Aufeinanderpressen zweier derartiger Meßflächen die erwähnten Saugeffekte vermieden, so daß die beim Auseinanderziehen der beiden Gummiproben gemessene maximale Abzugskraft ein direktes und in hohem Grade reproduzierbares Maß für die Grünklebrigkeit der in Frage stehenden Gummimischungen darstellt.

In vorteilhafter Ausgestaltung der Erfindung sind zusätzlich die in Anspruch 2 angegebenen Verfahrensschritte vorgesehen. Danach wird die konvexe Wölbung der Meßflächen der Gummiproben dadurch erreicht, indem auf die in einem Probenhalter eingelegte Probe eine Lochblende einer vorbestimmten Form und Größe aufgelegt und beispielsweise durch Festschrauben festgelegt wird. Durch dieses Niederdrücken der Lochblende auf der Probe wird die Gummimischung durchgedrückt und wölbt sich durch das Loch der Lochblende nach außen. Der durch das Loch der Lochblende herausragende Anteil der Gummiprobe stellt somit die Meßfläche der Probe dar.

Um eine möglichst gute Reproduzierbarkeit zu gewährleisten, wird gemäß dem Merkmal von Anspruch 3 vor dem Aufeinanderpressen der gewölbten Meßflächen eine Lochfolie zwischen die beiden Meßflächen gelegt, wodurch eine besonders gute Konstanz der Haftfläche zwischen den beiden Gummimischungen erzielt wird.

Weitere vorteilhafte Ausgestaltungen des erfindungsgemäßen Verfahrens sind in den sich anschließenden Unteransprüchen beschrieben.

Zur weiteren Ausgestaltung der Aufgabe wird erfindungsgemäß eine Vorrichtung zum Bestimmen der Grünklebrigkeit von Gummimischungen mit den Merkmalen des Anspruchs 9 vorgeschlagen. Diese Vorrichtung ist insbesondere zum Durchführen des erfindungsgemäßen Verfahrens geeignet. Die Vorrichtung ist einfach und kostengünstig herstellbar und gestattet das Herstellen von erfindungsgemäßen Gummimischungsproben mit konvex gewölbter Meßfläche mit einer zuverlässigen und gut reproduzierbaren Konstanz der Größe der Meßfläche.

Die Erfindung ist in der beiliegenden Zeichnung anhand eines Ausführungsbeispiels dargestellt und wird im folgenden unter Bezugnahme auf die Zeichnung ausführlich beschrieben.
- Figur 1: zeigt eine perspektivische schematische Darstellung einer erfindungsgemäßen Vorrichtung zum Bestimmen der Grünklebrigkeit von Gummimischungen,
- Figur 2: zeigt in seitlicher Schnittdarstellung einen Probenhalter mit Lochblende und eingelegter Mischungsprobe zur Verwendung in der Vorrichtung gemäß Figur 1 und
- Figur 3: veranschaulicht in seitlicher schematischer Darstellung das Aufeinanderdrücken von erfindungsgemäß konvex gewölbten Meßflächen mit einer zwischengelegten Lochfolie.

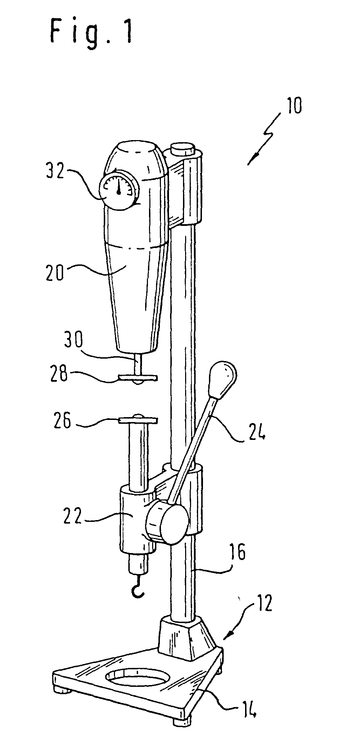

Figur 1 zeigt eine erfindungsgemäße Vorrichtung 10 zum Bestimmen der Grünklebrigkeit von Gummimischungen in perspektivischer Darstellung. Die Vorrichtung 10 umfaßt einen Prüfständer 12 mit einem Standfuß 14 und einer sich von dem Standfuß 14 vertikal nach oben erstreckenden Rundstange 16. Die Rundstange 16 dient zur Halterung eines Druck- und Zugkraftmeßgeräts 20, 22, das eine in der Nähe des oberen Endes der Rundstange 16 angebrachte Druck- und Zuglehre 20 umfaßt. Unterhalb der Druck- und Zuglehre 20 ist an der Rundstange 16 ein Gegenstück 22 angeordnet. Das Gegenstück 22 ist mittels eines Zughebels 24 entlang der Rundstange 16 nach oben und unten verstellbar. In seiner Ruhestellung liegt das Gegenstück 22 beabstandet zu der Druck- und Zuglehre 20. Bei dem Zughebel 24 handelt es sich vorteilhafterweise um einen verzögerungsfrei wirkenden Zughebel für schnelle Messungen.

Das Druck- und Zugkraftmeßgerät 20, 22 umfaßt des weiteren einen unteren und einen oberen Probenhalter 26, 28, die zueinanderweisend und konzentrisch zueinander angeordnet sind. Der untere Probenhalter 26 ist dem Gegenstück 22 zugeordnet und an diesem befestigt. Der obere Probenhalter 28 ist der Druck- und Zuglehre 20 zugeordnet und mit deren Meßgestänge 30 verbunden.

Durch Betätigen des Zughebels 24 kann das Gegenstück 22 entlang der Rundstange 16 so weit nach oben verstellt werden, daß der untere Probenhalter 26 des Gegenstücks 22 den oberen Probenhalter 28 der Druck- und Zuglehre 20 berührt und beaufschlägt. Abhängig von der Kraft, mit der der Zughebel 24 betätigt wird, wird der obere Probenhalter 28 durch die Beaufschlagung mit dem unteren Probenhalter 26 mehr oder weniger weit mit dem Meßgestänge 30 nach oben gedrückt. Diese Auslenkung des oberen Probenhalters 28 und des Meßgestänges 30 wird in der Druck- und Zuglehre 20 in eine Kraftanzeige an einem Anzeigeinstrument 32 umgesetzt.

Wird der Zughebel 24 wieder zurückbewegt und in der Gegenrichtung über seine Ausgangsstellung hinaus gedrückt, so senkt sich der untere Probenhalter 26 und mit ihm der obere Probenhalter 28 ab. Da die auf den Probenhaltern 26, 28 als Proben angebrachten Gummimischungen aneinander haften, wird der obere Probenhalter 28 über seine Ruhestellung hinaus nach unten ausgelenkt. Diese Auslenkung erfolgt solange, bis die beiden Gummimischungen voneinander abgezogen sind und wird an dem Anzeigeinstrument 32 als Zugkraft angezeigt.

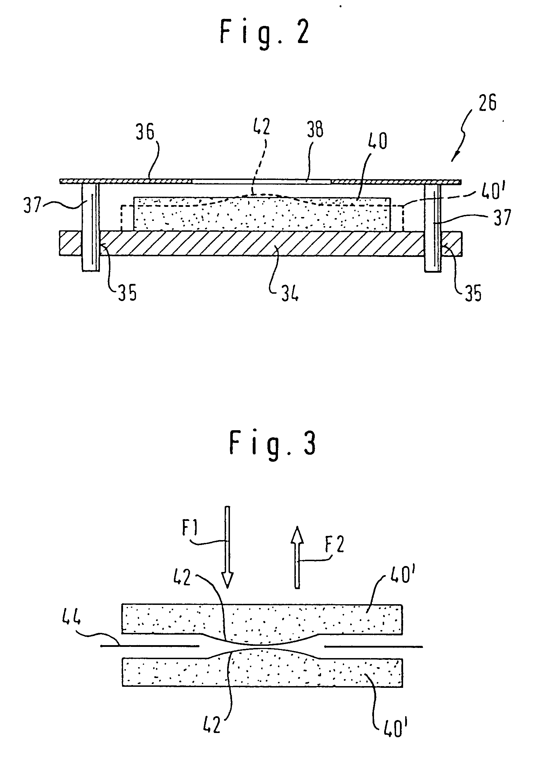

Figur 2 zeigt zur beispielhaften Erläuterung in seitlicher Schnittdarstellung den unteren Probenhalter 26 des Druck- und Zugkraftmeßgeräts 12 der Figur 1. Der obere Probenhalter 28 verfügt über im wesentlichen den gleichen Aufbau. Der Probenhalter 26 besteht aus einem im wesentlichen ebenen rechteckigen oder quadratischen Probentisch 34, auf dem eine Probe 40 einer Gummimischung, deren Grünklebrigkeit zu bestimmen ist, angeordnet werden kann. Oberhalb der Probe 40 ist eine Lochblende 36 mit einem zentrisch angeordneten Loch 38 vorgesehen. Die Lochblende 36 verfügt über mehrere in ihrem Randbereich angeordnete, senkrecht abstehende Stifte 37, mit denen sie zur Ausrichtung und Führung in entsprechend vorgesehene Bohrungen 35 des Probentischs 34 eingesetzt werden kann. Vorteilhafterweise sind vier jeweils in den Eckbereichen der Lochplatte 36 angeordnete Stifte 37 vorgesehen. Die Stifte 37 und die Bohrungen 35 sind so bemessen, daß die Stifte 37 mit nur geringem Spiel in die Bohrungen 35 eingesetzt und dort geführt werden können.

Die Probe 40 wird auf dem Probentisch derart angeordnet, daß sie das Loch 38 der Lochblende 36 vollständig ausfüllt und sich an allen Seiten ausreichend weit über die Ränder des Lochs 38 hinaus erstreckt. Danach wird die Lochblende 36 abgesenkt und gegen die Probe 40 gedrückt, so daß diese durchgedrückt wird. Im Bereich des Lochs 38 entsteht durch dieses Zusammendrücken der Probe 40 eine Wölbung 42. Die Lochblende 36 wird in diesem niedergedrückten Zustand festgelegt, beispielsweise durch (nicht dargestellte) Flügelmuttern, die auf einem auf den Stiften 37 vorgesehenen Schraubgewinde aufgesetzt und festgezogen werden können. Durch das Niederdrücken der Lochblende 36 nimmt die Probe 40 die in der Figur 2 gestrichelt dargestellte Form 40' an. Das erfindungswesentliche Merkmal besteht in der durch das Vorhandensein des Lochs 38 erzeugten konvexen Wölbung der Probe 40', wobei die Lochblende 36 so weit niedergedrückt wird, daß die Wölbung nach oben durch das Loch 38 hindurch über die Lochblende 36 hinausragt und als Meßfläche 42 der Probe 40' dienen kann.

Figur 3 veranschaulicht das der Erfindung zugrundeliegende Meßprinzip durch Aufeinanderdrücken und anschließendes Auseinanderziehen zweier Proben 40', die jeweils über eine erfindungsgemäß gewölbte Meßfläche 42 verfügen. Die Wölbung der Meßfläche 42 kann dabei beispielsweise auf die vorstehend erläuterte Art und Weise erzeugt worden sein. Zur Vereinfachung der Darstellung sind in Figur 3 nur die Proben der Gummimischungen ohne die jeweiligen Probenhalter dargestellt.

Die beiden Proben 40' bestehen aus jeweils einer Gummimischung, deren Grünklebrigkeit bestimmt werden soll. Dabei kann die Gummimischung bei beiden Proben identisch sein, jedoch ist es auch möglich, zwei verschiedene Gummimischungen auszumessen.

Die Proben 40' sind zur Durchführung des erfindungsgemäßen Verfahrens mit ihren konvex gewölbten Meßflächen 42 konzentrisch gegenüberliegend und zueinanderweisend angeordnet. Danach werden die beiden Proben 40' während einer bestimmten Zeit mit einer bestimmten Kraft gegeneinander gedrückt. Diese Druckkraft ist durch den nach unten weisenden Pfeil F1 der Figur 3 veranschaulicht. Anschließend werden die beiden Proben 40' entgegengesetzt der Druckkraft F1 bis zu einer maximalen Abzugskraft zugbelastet, so daß sie sich voneinander lösen. Diese maximale Abzugskraft ist durch den nach oben weisenden Pfeil F2 der Figur 3 veranschaulicht. In der Regel wird die maximale Abzugskraft kleiner sein als die Druckkraft, mit der die beiden Proben 40' zusammengedrückt wurden, weshalb der Pfeil F2 kürzer ist als der Pfeil F1.

Durch die erfindungsgemäße konvexe Wölbung der Meßflächen 42 werden bei der Bestimmung der Grünklebrigkeit von Gummimischungen Meßfehler verhindert, die bei herkömmlichen ebenen Proben durch Saugeffekte hervorgerufen werden. Erfindungsgemäß werden somit zuverlässige und reproduzierbare Meßwerte erzielt. Um die Reproduzierbarkeit der Messung noch weiter zu verbessern, kann zwischen den beiden Proben 40' noch eine Lochfolie 44 vorgesehen sein, die weiter dazu beiträgt, die tatsächliche Meßfläche, d.h. die Fläche, über die die beiden gewölbten Meßflächen 42 tatsächlich aneinander haften, konstant zu halten. Ein geeignetes Material für die Lochfolie 44 ist beispielsweise Polyester.

Bei der Vorbereitung der Proben ist zur Gewährleistung einer guten Reproduzierbarkeit darauf zu achten, daß die Proben auf Raumtemperatur abgekühlt sind und daß sich auf der gewölbten Meßfläche weder Staub noch Schmutz oder Feuchtigkeit befindet. Die Meßflächen 42 sollten insbesondere nicht mit der Hand berührt werden, da das bei einer Berührung abgeschiedene Hautfett zu Meßfehlern führt. Gegebenenfalls sind die Meßflächen mit einer glatten Folie abzudecken.

Damit eine gute Wölbung der Meßfläche erzielt werden kann, sollten die Proben weder zu dick noch zu dünn sein. Zu dicke Proben werden weiter gespalten, wobei die Meßfläche dazu mit einer glatten Folie abgedeckt wird. Zu dünne Proben werden auf ein Trägermaterial, beispielsweise auf Fell dubliert. Gewebe werden vorteilhafterweise über Kreuz gemessen, textiles Gewebe wird auf Fell dubliert und Stahlcord wird nicht dubliert. Kernreiter werden mit sich selbst so dubliert, daß sich eine gleiche Probendicke ergibt.

Als vorteilhaft hat sich für die Gummmimischungsproben eine Dicke von maximal 15 mm erwiesen bei einer Fläche von etwa 40 x 40 mm². Die Lochblende sollte ausreichend dünn sein, so daß die Wölbung der Probe problemlos durch das Loch über die Lochblende hinausragen kann, jedoch auf der anderen Seite ausreichend stabil sein, damit keine Verformungen auftreten. Die Dicke der Lochblende beträgt etwa 1,5 mm, das Loch in der Lochblende ist im wesentlichen kreisförmig mit einem Durchmesser von 1,5 cm, was einer Meßfläche von etwa 1,77 cm² entspricht.

Zur Durchführung der Messung wird das Meßgerät auf den Nullpunkt eingestellt und mit dem Zughebel wird der Druck zügig, d.h. während etwa 1 Sekunde, auf 50 N erhöht. Dieser Druck von 50 N wird 10 Sekunden lang gehalten, danach wird zügig, wiederum etwa 1 Sekunde, auf Null entlastet und etwas langsamer bis zur maximalen Abzugskraft zugbelastet (etwa 2 Sekunden). Die hier angegebenen Werte für den Anpreßdruck und die Anpreß-, Abzugs- und Haltezeiten haben sich in der Praxis als gut einhaltbar erwiesen. Natürlich können auch davon abweichende Werte vorgesehen werden, wobei es sich versteht, daß zu Zwecken der Vergleichbarkeit der Grünklebrigkeit von Gummimischungen über einen längeren Zeitraum diese Werte sich nicht mehr ändern sollten. Die Grünklebrigkeit gemäß der Klebkraftmethode HOCK entspricht dann der gemessenen maximalen Abzugskraft, bei der sich die Probenflächen voneinander lösen. Um den Meßvorgang besser nachvollziehen zu können, ist es vorteilhaft, an das Druck- und Zugkraftmeßgerät eine Einrichtung zum Aufzeichnen eines Kraft-Zeit-Meßdiagramms anzuschließen.

Insbesondere eignet sich die erfindungsgemäße Vorrichtung dazu, daß der manuelle Teil der Vorrichtung durch eine automatische, spindelgetriebene Einheit ersetzt wird. Die vorgeschriebenen Anpreß-, Abzugs-und Haltezeiten können dabei exakt eingehalten werden, so daß eine noch bessere Genauigkeit und Reproduzierbarkeit der Meßwerte erzielt wird.

Die hohe Reproduzierbarkeit der erfindungsgemäß erzielten Meßwerte wurde anhand von Versuchen belegt. Dabei haben vier Testpersonen bei jeweils drei durchgeführten Messungen und gleichen Proben die nachfolgend aufgeführten Meßwerte erzielt. Ebenfalls angegeben sind die Meßungenauigkeiten und die Standardabweichungen.

| **Testperson** | **a** | **b** | **c** | **d** |
|---|---|---|---|---|
| Wert 1 [N/1,8 cm²] | 40 | 42 | 39 | 41 |
| Wert 2 [N/1,8 cm²] | 43 | 39 | 43 | 40 |
| Wert 3 [N/1,8 cm²] | 39 | 40 | 42 | 42 |
| **Mittelwert** | **40,7** | **40,3** | **41,2** | **41,0** |

| | **Einzelmessungen** | **Mittelwerte** |
|---|---|---|
| Anzahl der Meßwerte: | 12 | 4 |
| Mittelwert gesamt | 40,8 | 40,8 |
| Vertrauensbereich (90%) | ± 0,8 | ± 0,5 |
| Standardabweichung | 1,5 | 0,4 |

Angesichts der durch diese Versuche belegten hohen Auflösung besitzt schon eine Einzelmessung gemäß dem erfindungemäßen Verfahren eine sehr gute Aussagekraft. Um einen zuverlässigen Meßwert zu erzielen, empfiehlt es sich, den Mittelwert aus drei Messungen zu bilden.

## Patentansprüche

1. Verfahren zum Bestimmen der Grünklebrigkeit mit folgenden Schritten:
1. Vorsehen zweier Proben (40') einer oder verschiedener Gummimischungen, deren Grünklebrigkeit bestimmt werden soll, wobei die Proben (40') jeweils ein Meßfläche (42) vorbestimmter Größe mit einer konvexen Wölbung aufweisen,
2. deckungsgleiches Aufeinanderlegen der Meßflächen (42) der beiden Proben (40'),
3. Beaufschlagen der aufeinandergelegten Meßflächen (42) mit einem vorbestimmten Druck während einer vorbestimmten Zeit,
4. Trennen der beiden Proben (40') durch Abziehen mit konstanter Geschwindigkeit und Bestimmen der maximalen Abzugskraft als Maß für die Grünklebrigkeit.

2. Verfahren nach Anspruch 1, **dadurch gekennzeichnet**, daß Schritt 1 sich durch folgende Teilschritte zusammensetzt:
1.1 Vorsehen zweier flächiger Proben (40) von Gummimischungen, deren Grünklebrigkeit bestimmt werden soll,
1.2 Einlegen jeder Probe (40) in einen Probenhalter (26, 28),
1.3 Auflegen einer Lochblende (36) auf jede Probe (40), und
1.4 Festlegen der Lochblende (36) derart, daß die Probe (40) durchgedrückt wird und sich durch das Loch (38) der Lochblende (36) nach außen wölbt.

3. Verfahren nach Anspruch 1 oder 2, **dadurch gekennzeichnet**, daß zum Konstanthalten der Meßfläche vor dem deckungsgleichen Aufeinanderlegen der Proben (40') zwischen den Proben (40') eine Lochfolie (44) vorgesehen wird.

4. Verfahren nach einem der Ansprüche 1 bis 3, **dadurch gekennzeichnet**, daß die Meßfläche (42) im wesentlichen kreisrund ist.

5. Verfahren nach Anspruch 4, **dadurch gekennzeichnet**, daß der Durchmesser der Meßfläche 1,5 cm beträgt.

6. Verfahren nach einem der vorhergehenden Ansprüche, **dadurch gekennzeichnet**, daß die Meßflächen (42) während 10 Sekunden mit einem Druck von 50 N beaufschlagt werden.

7. Verfahren nach einem der vorstehenden Ansprüche, **dadurch gekennzeichnet**, daß zum Kontrollieren und Messen der Druck-und Zugkraft ein Druck- und Zugkraftmeßgerät (20, 22) vorgesehen ist.

8. Verfahren nach Anspruch 7, **dadurch gekennzeichnet**, daß eine Einrichtung zum Aufzeichnen eines Kraft-Zeit-Meßdiagramms vorgesehen ist.

9. Vorrichtung zum Bestimmen der Grünklebrigkeit von Gummimischungen, insbesondere gemäß einem Verfahren nach einem der Ansprüche 1 bis 8, mit zwei jeweils eine Lochblende (36) aufweisenden Probehaltern (26, 28) zur Aufnahme einer Probe (40) einer Gummimischung, deren Grünklebrigkeit zu bestimmen ist, wobei durch Auflegen und Festziehen der Lochblende (36) auf der Probe (40) eine Meßfläche (42) mit konvex gewölbter Oberfläche erzeugbar ist und wobei die Probehalter (26, 28) senkrecht zu der Meßfläche (42) gegeneinander verstellbar in einem Druck- und Zugkraftmeßgerät (20, 22) angeordnet sind, das eine Druck- und Zuglehre (20) zum Messen der zum Zusammendrükken und Auseinanderziehen der Meßflächen (42) aufgewendeten Druck- oder Zugkraft aufweist.

10. Vorrichtung nach Anspruch 9, **dadurch gekennzeichnet**, daß eine Einrichtung zum Aufzeichnen eines Kraft-Zeit-Meßdiagramms vorgesehen ist.

## Claims

1. A method for determining the green tack of rubber blends comprising the following steps:
1. Providing two specimens (40') of one or differing rubber blends, the green tack of which is to be determined, each of said specimens (40') comprising a measurement surface area (42) of a predetermined size and having a convex curvature,
2. Locating said measurement surface areas (42) of said two specimens (40') flush one on the other,
3. Subjecting said measurement surface areas (42) placed one on the other to a predetermined pressure for a predetermined period of time,
4. Parting said two specimens (40') by peeling at a constant rate and determining the maximum parting force as a measure of the green tack.

2. The method as set forth in claim 1, wherein said step 1 involves the following sub-steps:
1.1 Providing two surface area specimens (40') of rubber blends, the green tack of which is to be determined.
1.2 Inserting each specimen (40) in a specimen mount (26, 28),
1.3 Applying an orifice disk to each said specimen (40), and
1.4 Defining said orifice disk (36) such that said specimen (40) is forced therethrough to bulge outwardly from the orifice (38) of said orifice disk (36).

3. The method as set forth in claim 1 or claim 2, wherein for maintaining said measurement surface area (42) constant an orifice film (44) is provided between said specimens (40') prior to placement of said specimens (40') flush one on the other.

4. The method as set forth in any of the claims 1 to 3, wherein said measurement surface area (42) is substantially circular.

5. The method as set forth in claim 4, wherein the diameter of said measurement surface area (42) is 1.5 cm.

6. The method as set forth in any of the preceding claims, wherein said measurement surface areas (42) are subjected to a pressure of 50 N for 10 seconds.

7. The method as set forth in any of the preceding claims, wherein a combination push/pull dynamometer (20, 22) is provided for controlling and measuring the force in pressure and tension.

8. The method as set forth in claim 7, wherein a means for plotting a force/time graph is provided.

9. A device for determining the green tack of rubber blends, more particularly in accordance with the method as set forth in any of the claims 1 to 8, comprising two specimen mounts (26, 28) each incorporating an orifice disk (36) for mounting a specimen (40) of a rubber blend, the green tack of which is to be determined, whereby by placing and tightening said orifice disk (36) on said specimen (40) a measurement surface area (42) having a convex curved surface is producible, said specimen mounts (26, 28) being arranged vertically adjustable to said measurement surface area (42) in a combination push/pull dynamometer (20, 22) having a push/pull gauge (20) for measuring the force of pressure or tension applied to compress and part said measurement surface areas (42) respectively.

10. The device as set forth in claim 9, wherein a means for plotting a force/time graph is provided.

## Revendications

1. Procédé pour déterminer l'adhésion à vert, comprenant les étapes suivantes :
1) on prévoit deux éprouvettes (40') d'un ou de plusieurs mélanges de caoutchouc dont on veut déterminer l'adhésion à vert, les éprouvettes (40') présentant chacune une surface de mesure (42) de taille prédéterminée avec un bombement convexe,
2) on superpose en coïncidence les surfaces de mesure (42) des deux éprouvettes (40'),
3) on sollicite les surfaces de mesure superposées (42) par une pression prédéterminée pendant une période prédéterminée,
4) on sépare les deux éprouvettes (40') par tirage à vitesse constante et on détermine la force de tirage maximale comme valeur pour l'adhésion à vert.

2. Procédé selon la revendication 1, **caractérisé en ce que** l'étape 1 se compose des étapes partielles suivantes :
1.1 on prévoit deux éprouvettes plates (40) de mélanges de caoutchouc dont on veut déterminer l'adhésion à vert,
1.2 on pose chaque éprouvette (40) dans un porte-éprouvette (26, 28),
1.3 on pose une plaque trouée (36) sur chaque éprouvette (40), et
1.4 on fixe la plaque trouée (36) de telle sorte que l'éprouvette (40) est est mise sous pression et se bombe vers l'extérieur à travers le trou (38) de la plaque trouée (36).

3. Procédé selon l'une ou l'autre des revendications 1 et 2, **caractérisé en ce que** pour maintenir constante la surface de mesure avant la superposition en coïncidence des éprouvettes (40'), on prévoit un film troué (44) entre les éprouvettes (40').

4. Procédé selon l'une quelconque des revendications 1 à 3, **caractérisé en ce que** la surface de mesure (42) est sensiblement circulaire.

5. Procédé selon la revendication 4, **caractérisé en ce que** le diamètre de la surface de mesure s'élève à 1,5 cm.

6. Procédé selon l'une quelconque des revendications précédentes, **caractérisé en ce qu**'on sollicite les surfaces de mesure (42) par une pression de 50 N pendant 10 secondes.

7. Procédé selon l'une quelconque des revendications précédentes, **caractérisé en ce qu**'on prévoit un appareil de mesure de force de pression et de traction (20, 22) pour contrôler et mesurer la force de pression et de traction.

8. Procédé selon la revendication 7, **caractérisé en ce que** l'on prévoit un dispositif pour enregistrer un diagramme de mesure force/temps.

9. Dispositif pour déterminer l'adhésion à vert de mélanges de caoutchouc, en particulier par un procédé selon l'une quelconque des revendications 1 à 8, comportant deux porte-éprouvettes (26, 28) présentant chacun une plaque trouée (36) pour recevoir une éprouvette (40) d'un mélange de caoutchouc dont l'adhésion à vert est à déterminer, dans lequel une surface de mesure (42) avec une superficie bombée convexe peut être produite par déposition et serrage de la plaque trouée (36) sur l'éprouvette (40), et dans lequel les porte-éprouvettes (26, 28) sont agencés dans un appareil de mesure de force de pression et de traction (20, 22) de façon réglable l'un par rapport à l'autre perpendiculairement à la surface de mesure (42), ledit appareil comprenant une jauge de pression et de traction (20) pour mesurer la force de pression ou de traction appliquée pour comprimer ou écarter les surfaces de mesures (42).

10. Dispositif selon la revendication 9, **caractérisé en ce qu**'il est prévu un dispositif pour enregistrer un diagramme de mesure force/temps.
